(19) Europäisches Patentamt
European Patent Office
Office européen des brevets

(11) EP 4 371 556 B1

(12) **EUROPEAN PATENT SPECIFICATION**

(45) Date of publication and mention of the grant of the patent:
**05.08.2026 Bulletin 2026/32**

(21) Application number: **22207541.8**

(22) Date of filing: **15.11.2022**

(51) International Patent Classification (IPC):
***A61K 9/00*** *(2006.01)* ***A61K 9/08*** *(2006.01)*
***A61K 9/19*** *(2006.01)* ***A61K 47/20*** *(2006.01)*
***A61K 47/26*** *(2006.01)* ***A61K 38/00*** *(2006.01)*
***A61K 38/08*** *(2019.01)*

(52) Cooperative Patent Classification (CPC):
**A61K 9/19; A61K 9/0043; A61K 9/0078; A61K 9/08; A61K 38/085; A61K 47/26**

(54) **ANGIOTENSIN(1-7) PHARMACEUTICAL COMPOSITIONS FOR INHALATION**

PHARMAZEUTISCHE ANGIOTENSIN(1-7)-ZUSAMMENSETZUNGEN ZUR INHALATION

COMPOSITIONS PHARMACEUTIQUES D'ANGIOTENSINE(1-7) POUR INHALATION

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(43) Date of publication of application:
**22.05.2024 Bulletin 2024/21**

(73) Proprietor: **Explicat Pharma GmbH**
**85662 Hohenbrunn (DE)**

(72) Inventors:
- **WEILAND-WAIBEL, Andrea**
  **85662 Hohenbrunn (DE)**
- **HETTINGER, Birgit**
  **82184 Gröbenzell (DE)**
- **LÖFFLER, Stefan**
  **83052 Bruckmühl (DE)**

(74) Representative: **Becker, Eberhard**
**Becker Kurig & Partner**
**Patentanwälte mbB**
**Bavariastraße 7**
**80336 München (DE)**

(56) References cited:
**EP-A1- 2 991 663** **WO-A1-2009/056651**
**WO-A1-2022/094025**

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 371 556 B1

## Description

### Technical Field

The present invention relates to improved formulations comprising Angiotensin(1-7) for pharmaceutical use, and particularly to formulations comprising Angiotensin(1-7) for administration by inhalation, preferably by use with a nebuliser. The present invention therefore relates to pharmaceutical compositions comprising Angiotensin(1-7) in which Angiotensin(1-7) is present in a solubilized form, and the use of such formulations in the field of medicine. The formulations have therapeutically and commercially useful concentrations of Angiotensin(1-7). Administration of the compositions provides Angiotensin(1-7) to target organs for local or systemic action. Therefore, the present invention also relates to the pharmaceutical compositions for use in a method of treating local or systemic conditions which benefit from administration of Angiotensin(1-7).

### Background

The renin-angiotensin system (RAS), a portion of which is schematically illustrated in Figure **1A,** is a hormonal system regulating, *inter alia,* blood pressure and systemic vascular resistance. Key peptides involved in the RAS regulatory functions are the octapeptide Angiotensin II (ATII, **100,** SEQ. ID. N. 1) and the heptapeptide Angiotensin(1-7) (AT1-7, **105,** SEQ. ID. N. 2, chemical formula shown below).

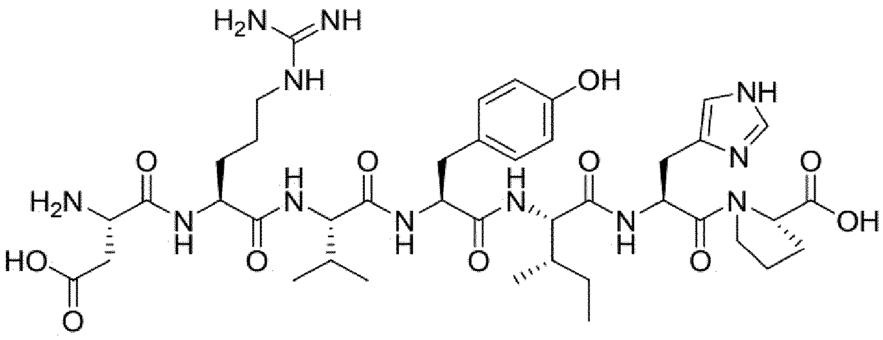

ATII **100** and AT1-7 **105** are known to reciprocally balance their associated biological effects, where, for example ATII **100** acts on the nuclear androgen receptor (AR1, **110)** as vasoconstrictor, inflammatory, proliferating agent and AT1-7 **105** acts on the G-coupled receptor MAS1 **(115)** as vasodilator; anti-inflammatory, anti-proliferating agent. Biochemically, ATII **100** is obtained from the decapeptide Angiotensin I (ATI, **120,** SEQ. ID. N. 3) by action of the angiotensin-converting enzyme (ACE, **130),** a peptidase which cleaves two amino acid residues (illustrated as fragment **142)** from ATI **120** to generate ATII **100.** Another amino acid residue **146** is cleaved from ATII **100** by another peptidase, the angiotensin-converting enzyme 2 (ACE2, **135)** to form AT1-7 **105.** Alternatively, AT1-7 **105** may be generated directly from ATI **120** by action of prolyl-endopeptidases and/or neutral-endopeptidases. ACE **130** may also act on AT1-7 **105** to cleave a further dipeptide fragment **146,** leaving a pentapeptide fragment **148,** commonly referred to as AT1-5 **150** (SEQ. ID. N. 4).

The RAS is active both in the circulatory system, as well as in various organs, which have developed their own specific RAS; see, e.g., Holappa et al., The Open Ophthalmology Journal, 2017, 11, 122-142. In view of its regulatory action, administration of AT1-7 **105** and derivatives thereof has been suggested to treat a variety of conditions such as cardiovascular diseases involving arterial hypertension, intraocular pressure, wounds, burns, erythema, tumors, diabetes mellitus, sperm mobility, nephropathy, gastrointestinal disorders, gynaecological disorders, renal insufficiency, angiogenesis, and alopecia, amongst others; see, e.g., EP 2 356 995 A2, EP 1 450 842 B1, EP 2 264 048 B1, WO 2007/000036 A2 (all by Santos, R.A.S. et al.); DE 28 46 200 A1; WO 01/55176 A2; Kuipers et al., Peptides, 2019, 112, 78-84; Mascolo et al., Frontiers in Pharmacology, 2021, 12, 667254; Nozato et al., Clinical Science, 2019, 133, 2005-2018; Vaajanen et al., Investigative Ophthalmology and Visual Science, 2008, 49, 2557-2562.

Referring to Figure **1A** and **1B,** in the past two years, COVID-19 has emerged as a life-threatening condition caused by infection with the coronavirus SARS-CoV-2 **160,** as the wild-type virus or one of its subsequently-evolved variants. It has been shown that subunits of the spike proteins of SARS-CoV-2 have high affinity for ACE2 receptors **135,** which ACE2 receptors **135** are used as entry point within the cell and are heavily downregulated following infection see, e.g., Zhou et al., Nature, 2020, 579, 270-273; Chatterjee, B. and Thakur, S.S., RSC Advances, 2020, 10, 39808-39813. A similar mechanism was already observed in the case of SARS-CoV infection back in 2005, see, e.g., Kuba et al., Nature Medicine, 2005, 11, 875-879. As a consequence of the downregulation of ACE2 **135,** ATII **100** tends to accumulate in the system, exercising its vasoconstrictory and inflammatory action, possibly causing severe symptoms and conditions, such

as acute lung injury, without production of the balancing factor AT1-7 **105.**

If, as illustrated in Figure **1C,** a system with reduced expression and/or activity of ACE2 **135** is externally supplied with AT1-7 **105,** the negative effects caused by the excess of ATII **100** is at least partially compensated, reducing the likelihood of an adverse course of the disease. Administration of AT1-7 **105** or its analogues to alleviate symptoms in patients affected by SARS-CoV-2 has been suggested, for example, in Liu et al., J. Clin. Lab. Anal., 2021, 35: e23789, Ni et al., Critical Care, 2020, 24:422, and in WO 2021/183863 A1. A list of clinical studies investigating administration of AT1-7 **105** for the treatment of COVID-19 is available, e.g., in Mascolo et al., Frontiers in Pharmacology, 2021, 12, 667254. As SARS-CoV-2 affects principally the respiratory system of the patient, with the virus localizing particularly in the lungs and upper respiratory tract, administration of AT1-7 **105** directly to the affected areas, e.g., via inhalation, would constitute an effective course of action for treatment as well as prevention of adverse events. Similar hypotheses have been formulated in the literature, for example by Peiró C. and Moncada S., Circulation, 2020, 141, 1665-1666; Magalhaes, G. S., et al., Frontiers in Physiology, 2020, 11, 73.

More generally, it is believed that the pharmacodynamic target for AT1-7 **105** is the MAS receptor **115,** or heterodimers of MAS receptors **115.** In fact, intraperitoneal injection of AT1-7 **105** was shown to inhibit LPS-induced early pulmonary fibrosis (Cao Y. et al., Laboratory Investigation, 2019, 99, 1770-1783), while intravenous administration of AT1-7 **105** was successful in the treatment of preclinical models of acute lung injury (Supé et al., British Journal of Pharmacology, 2016, 173, 1618-1628; Supé, Bestimmung des optimalen Zeitfensters für die therapeutische Anwendung von Angiotensin-(1-7) beim akuten Atemnotsyndrom bei der Ratte, Doctoral Dissertation, Freien Universität Berlin, 2018**).** Additional therapeutic applications of AT1-7 **105** and its analogues, including the treatment of pulmonary conditions, have been recently reviewed in Khajehpour S. and Aghazedeh-Habashi A., J. Pharmacol. Exp. Ther. , 2021, 377, 64-74.

Upon administration of AT1-7 formulations, a vasodilatory effect and an immunomodulatory action may be expected. For example, administration by inhalation could particularly benefit certain respiratory conditions, such as acute respiratory distress syndrome (ARDS), bronchial asthma, chronic obstructive pulmonary disease, cystic fibrosis, berylliosis, and sarcoidosis. As another example, topical administration of formulations of AT1-7 in the eyes of model animals has been shown to effectively reduce intraocular pressure, and could be considered to treat RAS-related conditions of the eye, such as glaucoma, diabetic retinopathy, age-related macular degeneration, and/or retinopathy in premature newborns. As a further example, topical administration to the nose may alleviate COVID-19 symptoms such as loss of smell and taste. Treatment of systemic conditions in view of the known action of AT1-7 **105** appears also possible.

WO 2009/055651 A1 discloses formulations comprising an angiotensin-virus-like particle conjugate and a stabilizer, wherein said stabilizer comprises a non-reducing disaccharide and a non-ionic surfactant.

WO 2022/094025 A1 discloses an aqueous isotonic composition comprising AT1-7 and a non-reducing sugar, whereby a surfactant, such as polysorbate may be included, and the pH is 5.

**Problems faced**

The majority of the literature documents cited above focusses on the provision of peptides analogues to AT1-7 **105** and their medical applications. While the possibility of administering AT1-7 **105** or its analogues via inhalation is mentioned in at least some of the documents referenced above, such references are substantially silent as to any suitable compositions for inhalation, in particular for nebulisation. EP 2 356 995 A2, which focuses on the provision of pharmaceutical compositions, suggests complexation with hydrophilic cyclodextrins or incorporation into biodegradable polymers, possibly further encapsulated in liposomes. The other cited references such as EP 1 450 842 B1 and EP 2 264 048 B1, provide similar indications, for example with respect to the combination of AT1-7 **105** or its analogues with cyclodextrins. In Magalhães et al., Immunobiology, 2020, 225, 151957, formulations for inhalation of AT1-7 **105** were prepared with hydroxypropyl-betacyclodextrin (HPβCD) in saline solution.

When the present inventors tested aqueous formulations of AT1-7 **105** in isotonic sodium chloride or borate buffer saline (pH adjusted with NaOH), formation of aggregates was observed by dynamic light scattering (DLS), as indicated in Table **1** below. The amount of AT1-7 **105** in Table 1 refers to the amount of AT1-7 as weighted to prepare the solutions for the experiments. Because in these experiments AT1-7 **105** was added as the triacetate salt, the effective amount of AT1-7 **105** is about 83% of the amount indicated (molecular weight AT1-7: 899.02 g/mol$^{-1}$; molecular weight acetic acid: 60.05 g mol$^{-1}$; 0.83= 899.02/(899.02+60.05*3)). Conditions for the dynamic light scattering measurements were as indicated further below in the description of the Examples. Turbidity was measured with a turbidimeter, the portable Turb® 430 by WRW/Xylem, according to the recommendation given in the European Pharmacopoeia, 10.0, 2.2.1 *"Clarity and degree of opalescence of liquids".*

**Table 1.** Measurement of AT1-7 **105** in isotonic saline or borate buffer solutions

| Formulation | AT1-7 (mg/mL) (as weighted) | pH | Turbity (NTU) | d50 (nm,intensity) | DLS, PDI |
|---|---|---|---|---|---|
| NaCl, 0.9% | 1.8 | 5.03 | 0.07 | 453.0 | 0.563 |
| | 6.0 | 5.02 | 0.21 | 317.6 | 0.386 |
| Borate buffer saline | 1.8 | 5.98 | 0.09 | 527.3 | 0.886 |
| | 6.0 | 6.04 | 0.35 | 211.7 | 0.224 |

All formulations, immediately after preparation and also after 2 and 5 days storage at 2-8°C show a salting out effect, likely due to aggregation of AT1-7. DLS analysis of the solutions after storage revealed an increase in aggregation, as determined by the increased area of the large-diameter (e.g., > 1 $\mu$m) peaks in the intensity distributions. The slightly higher turbidity measured for the higher concentrations indicates that the salting out (aggregate formation) is more pronounced at higher AT1-7 concentrations. As the tabulated turbidity values show, the effect is dependent on the amount of AT1-7 **105** used, and is thus attributable to AT1-7 **105.** Aggregation of AT1-7 **105** was further investigated by "dissolving" AT1-7 **105** in water for injection (WFI) and analysing such formulations via DLS. The results are reported in Table **2** below. Representative intensity distributions are reproduced in Figures **2A** and **2B.** In the distributions of Figures **2A** and **2B,** peaks corresponding to aggregates at higher particles diameters are clearly visible.

**Table 2.** DLS measurements of AT1-7 **105** in WFI

| AT1-7 (mg/mL) | 1 | 0.3 | 0.1 | 0.03* | 0.01* |
|---|---|---|---|---|---|
| Mean average diameter (nm, intensity) | 848.8 | 597.8 | 657.3 | 920.5 | 1639.6 |
| PDI | 0.407 | 0.288 | 0.319 | 0.429 | 0.719 |

In an attempt to solubilize the peptide, the effect of cyclodextrins was investigated. In particular, HP$\beta$CD, a cyclodextrin presented as preferable in most of the literature described above and used in composition for inhalation by Magalhães et al., was selected for testing. In Figure **3A** is plotted a representative intensity distribution obtained from DLS measurements performed on a formulation including isotonic HP$\beta$CD (25% m/V) and AT1-7 (1.8 mg/mL, as weighted, triacetate salt) in WFI, while Figure **3B** is the Higuchi-Connors plot resulting from a phase solubility analysis for AT1-7 **105** at different concentrations of HP$\beta$CD in isotonic saline solutions. The skilled person is familiar with Higuchi-Connors plots and how to obtain them, as described, e.g., in Higuchi T. and Connors, Advances in Analytical Chemistry and Instrumentation, 1965, 177-212. Unexpectedly, from the distribution of Figure **3A** aggregates are still revealed (cf. the peaks at >1000 nm) and, from the Higuchi-Connors plot of Figure **3B,** it appears that the tested cyclodextrin has substantially no solubilising effect on AT1-7 **105.** The peaks at >1000 nm in Figure **3A** may be attributed to aggregates at least because LSD measurements of isotonic HP$\beta$CD (25% m/V) in each of WFI and isotonic saline did not show any peaks above 10 nm, corresponding to the hydrodynamic radius of HP$\beta$CD.

Peptide formulations displaying aggregation behaviour as observed in the diffraction intensity distributions measured for AT1-7 **105** are known to have failed in clinical phase trials because of the aggregates triggering an immunogenic response when administered by inhalation. An objective of the present invention is therefore the provision of a pharmaceutical composition of AT1-7 **105** in which the active principle is well-solubilized, preferably without any sign of aggregation. Such pharmaceutical compositions would then be suitable for administering AT1-7 **105** to subjects in need thereof, preferably by inhalation, more preferably by inhalation following nebulisation.

**Summary**

Surprisingly, the present inventors found that pharmaceutical compositions as defined in claim 1 solve the above problems. These compositions may also be conveniently dried to obtain powders as defined in claim 7. The aqueous compositions or the dried powder may also be sold as a kit for inhalation, as set forth in claims 8 and 9. Also, the pharmaceutical compositions may be administered to human and/or veterinary subjects as a medicament for treatment of conditions requiring or benefitting from administration of AT1-7, as set forth in claim 10, for example by inhalation as set forth in claim 11, as topical ophthalmic treatment as set forth in claim 12, or as topical nasal treatment as set forth in claim 13.

The dependent claims set forth some preferred, beneficial embodiments.

## Brief description of the drawings

Without limiting the scope of the invention as defined in the appended claims, in the enclosed drawings:

Figures **1A** to **1C** are schematic diagrams illustrating some aspects of the renin-angiotensin-system, in physiological and pathological conditions;

Figures **2A** and **2B** are plots of intensity distributions measured by DLS for formulations of AT1-7 in water for injection;

Figure **3A** is a plot of the intensity distributions measured by DLS for formulations of AT1-7 and HPβCD in water for injection;

Figure **3B** is the Higuchi-Connors plot for the solubility of AT1-7 as a function of HPβCD concentration in isotonic saline solution;

Figure **4** is a schematic exemplar process flow for the preparation of a pharmaceutical composition according to some embodiments of the invention;

Figures **5A** to **5C** are plots of intensity distributions measured by DLS for formulations of AT1-7 in water for injection with different amounts of polyethylene (80) sorbitan monolaureate (PS80);

Figures **6A** to **6D** are plots of intensity distributions measured by DLS for formulations of AT1-7 in water for injection in presence of different osmolytes at isotonic concentration (Figure **6A:** NaCl; Figure **6B:** Glycine; Figure **6C:** Albumin; Figure **6D:** Trehalose);

Figures **7A** to **7C** are plots of intensity distributions measured by DLS for formulations of AT1-7 in water for injection in presence of different osmolytes at isotonic concentration and PS80 at 0.1 mass/V % (Figure **7A:** NaCl; Figure **7B:** Glycine; Figure **7C:** Trehalose);

Figure **8** is a plot of the intensity distribution measured by DLS for a formulation of AT1-7 in water for injection in presence of isotonic HPβCD and PS80 at 0.1 mass/V %.

Figure **9A** is a plot of the intensity distribution measured by DLS for concentrated formulations of AT1-7 (5 mg/mL, net), PS80 (0.3 % m/V), and trehalose (9.4 % m/V) in water for injection;

Figure **9B** is a plot comparing intensity distributions measured by DLS for formulations of AT1-7 (1 mg/mL, net), PS80 (0.03 % m/V), in absence (curve **300)** and presence (curve **302)** of trehalose (9.4 % m/V) in water for injection;

Figure **10** is a plot of fluorescence recovery traces measured to test permanence of the biological activity of AT1-7 following nebulisation.

## Detailed description

In one embodiment, it is provided a pharmaceutical composition for administration of AT1-7 **105,** preferably for inhalation, more preferably by nebulisation, which pharmaceutical composition includes AT1-7 **105** as active principle, and further includes a hydrophilic surfactant and a non-reducing sugar.

AT1-7 **105** is a heptapeptide with a sequence according to SEQ. ID. N. 2, namely Asp-Arg-Val-Tyr-Ile-His-Pro. AT1-7 **105** may be included in the pharmaceutical compositions as a pharmaceutically acceptable salt. For example, when prepared by solidphase synthesis, AT1-7 **105** may be initially obtained as the trifluoroacetate salt. Ion exchange may be performed to obtain AT1-7 **105** as a pharmaceutically acceptable salt. Exemplar pharmaceutically acceptable counterions are halides (chloride, bromide), acetate, formate, fumarate, maleate, adipate, ascorbate (preferably, L-ascorbate), benzoate, malate (preferably L-malate), mesylate, nitrate, oxalate, succinate, tartrate (D- or L- tartrate), hydrogen/di-hydrogen phosphate, hydrogen sulphate, sulphate, and tosylate. Preferably, acetate is selected as counterion.

AT1-7 **105** is included in the pharmaceutical composition so as to be delivered, upon nebulisation, in a therapeutic effective amount. For example, AT1-7 **105** may be included at a concentration of about 3.0 x $10^{-7}$ mol/mL to 3.0 x $10^{-5}$ mol/mL, preferably 5.0 x$10^{-7}$ mol/L to 1 x $10^{-5}$ mol/L, and more preferably 2 x $10^{-6}$ mol/mL to 8 x $10^{-6}$ mol/L, such as at about 5.6 x $10^{-6}$ mol/L. For example, AT1-7 **105** (as active principle, at net of the counterion) may be included at 0.27 mg/mL to 27 mg/mL, preferably 0.51 mg/mL to 9.0 mg/mL, more preferably 1.8 to 7.2 mg/mL, such as at about 5.0 mg/mL. For example,

when AT1-7 **105** is used as triacetate salt, about 83% of the weighted mass constitutes the active principle, so that to prepare a 5 mg/mL solution, about 6 mg of the peptide as the triacetate salt should be used. In some embodiments, the concentration of AT1-7 **105** may be selected so that upon nebulization of about 1.0 mL of solution, at least 1.0 mg of active principle is inhaled by the subject. Nebulization speed may be selected or indicated by the skilled person as a matter of routine. For example, the nebulization speed may be selected so that 1 mL of solution is inhaled in about 4.4 minutes.

In some embodiments, the concentration for nebulisation may be obtained by diluting the pharmaceutical composition with a suitable amount of dilution liquid, which dilution liquid may be identical to the pharmaceutical composition but for not comprising the active principle. Therefore, the pharmaceutical composition for nebulisation may have the active principle diluted with respect to the other components (e.g., surfactant, non-reducing sugar) with respect to the pharmaceutical composition for dilution. In some embodiments, the pharmaceutical composition and the dilution liquid may be provided as parts of a kit. For example, the kit may include a pharmaceutical composition comprising AT1-7 **105** at about 5.0 mg/mL (net of any counterion), and a dilution liquid to dilute the pharmaceutical composition to reach a concentration of AT1-7 **105** of about 1.0 mg/mL (net of counterion). In some alternative embodiments, the dilution factor for the pharmaceutical composition for dilution with respect to the pharmaceutical composition for nebulisation may be in the range from 2x to 10x, preferably in the range from 4x to 8x. In some alternative embodiments, the pharmaceutical composition may be provided already diluted at the intended concentration of use.

AT1-7 **105** being a small peptide, including charged residues as well as residues capable of hydrogen bonding with the solvent, it was rather surprising to encounter aggregation and precipitation in water to the extent observed. Without being bound to or limited by any theory, it is possible that the aggregation and precipitation observed for AT1-7 **105** may be caused by a small hydrophobic core formed by the three central residues, Val-Tyr-Ile. In view of the results presented herein, the solution found by the present inventors, effectively keeps AT1-7 **105** dissolved

In addition to the active principle, the pharmaceutical composition includes the hydrophilic surfactant. As hydrophilic surfactant, a polysorbate having a lipophilichydrophilic balance, HLB, value of 8 or more, preferably 10 or more, particularly preferably of 12 or more; and even more preferably of 15 or more (on a scale of 20) is used. So, for example, the HLB value of the surfactant may be in the range from 8 to 20, preferably in the range from 10 to 20, particularly preferably in the range from 12 to 20, and even more preferably in the range from 15 to 18. The HLB values referred here are the ones evaluated according to the calculation method reported in Gadhave A., International Journal of Science and Research, 2014, 3, 573-575, § 2.1, . In particular, for polyhydric alcohol fatty acid esters, the HLB value is calculated by the formula

$$HLB = 20\left(1 - \frac{S}{A}\right)$$

in which S is the saponification number of the ester and A is the acid number of the acid.

The hydrophilic surfactant is a polysorbate. Preferably, the hydrophilic surfactant is selected from polyoxyethylene (20) sorbitan monooleate (PS80) and polyoxyethylene (20) sorbitan monolaurate (PS20). Most preferably, the hydrophilic surfactant is polyoxyethylene (20) sorbitan monooleate (PS80).

The amount of hydrophilic surfactant is not particularly limited, and may be included in molar defect up to molar excess with respect to AT1-7 **105.** The molar ratio of the surfactant to the AT1-7 **105** is at least 0.1:1 (surfactant:AT1-7). Preferably, a molar ratio of AT1-7 to the hydrophilic surfactant may be in the range from 1:0.1 to 1:20. In solution for storage and dilution, more preferably, the molar ratio of AT1-7 to the hydrophilic surfactant may be in a range from 1:0.1 to 1:3, still more preferably in a range from 1:0.2 to 1:0.8, and most preferably, in a range from 1:0.3 to 1:0.6. For example, the molar ratio of AT1-7 **105** to the hydrophilic surfactant may be about 1:0.4. In a solution for nebulization, more preferably, the molar ratio of AT1-7 to the hydrophilic surfactant may be in a range from 1:0.5 to 1:15, still more preferably, 1:1 to 1:4, and most preferably in a range from 1:1.5 to 1:3. For example, the molar ratio of AT1-7 **105** to the hydrophilic surfactant may be about 1:2.

In some embodiments, the hydrophilic surfactant is included in the composition at a concentration at least equal to or, preferably, higher than, its critical micelle concentration (CMC). As known to the skilled person, the critical micelle concentration of the hydrophilic surfactant may be measured by determining the surface tension of a concentration series with a tensiometer. Without being bound to or limited by any theory, it is possible that the micelles formed by the hydrophilic surfactant interact with AT1-7 **105** to stabilize the peptide in solution, for example by tuning or disrupting the interactions between AT1-7 **105** with the material of the walls of the container and/or gas overlying the solution. Taking as example an aqueous solution only partially filling a glass vial, adsorption of AT1-7 **105** at the glass-water interface and/or at the water-air interface may trigger aggregation of AT1-7 **105,** which aggregation may be successfully disrupted by the addition of the hydrophilic surfactant.

The pharmaceutical composition further includes a non-reducing sugar as an osmolyte. The non-reducing sugar is included at its isotonic concentration. In the context of the present disclosure, the non-reducing sugar being included at

its isotonic concentration means that the non-reducing sugar is included in an adequate amount for the pharmaceutical composition (including all its components) to have an osmolarity in the range from 250 to 350 mosmol/Kg, more preferably in the range from 270 to 330 mosmol/Kg. Trehalose is used as the osmolyte. An exemplar isotonic concentrations range for is from 11 to 8% m/V (e.g.,9.4 m/V %) for trehalose. Sugar concentrations suitable to adjust the osmolarity of the pharmaceutical composition in the desired range can be easily determined with methods known to the skilled person, for example according to the protocol described in *European Pharmacopoeia,* 2.2.35. *Osmolality,* by the measurement of a colligative property (i.e., freezing-point depression) of the composition. Dedicated instruments such as freezing points osmometers by Knauer may be used.

The pharmaceutical composition is an aqueous solution. That is, in addition to the AT1-7 **105,** the hydrophilic surfactant, and the non-reducing sugar, water is included as a solvent. Limited amounts of pharmaceutically acceptable co-solvents may optionally be included to form suitable solvent systems, (e.g., ethanol/water 1/99 up to 5/95, v/v). The pH of the solution is on the slightly acidic side. The pharmaceutical composition has a pH in the range from 4.0 to 6.0, more preferably in the range from 4.5 to 5.5. In some embodiments, the pharmaceutical composition may have a pH of about 5.0, such as 5.0±0.1. In some embodiments, the pH of the composition may be adequately adjusted, for example, by acidification with a suitable acid, such as acetic acid. In some embodiments, the pH adjusting agent may be selected to exercise a buffering action at the target pH of the composition, and may be added to the composition before dissolving the active principle. In some embodiments, as pH adjusting agent, the conjugate acid of the counterion of the active principle may be used. In some embodiment, the pH is adjusted so as to be lower than the estimated isoelectric point of the active principle.

In some embodiments, the pharmaceutical composition may consist of the active principle, the hydrophilic surfactant, the non-reducing sugar and the solvent system (e.g., water for injection, optionally pH adjusted). Additional ingredients and excipients such as flavourings, colorants, binders, glidants, lubricants, preservatives, sweeteners, etc, would thus be excluded. For example, the pharmaceutical compositions may not include inorganic salts. In addition or in alternative, the pharmaceutical compositions may not include solubilising agents other than the hydrophilic surfactant and the non-reducing sugar. In particular, the pharmaceutical compositions do not include cyclodextrins. In fact, in the most preferred embodiments of the invention, each one of the pharmaceutical compositions disclosed throughout the description does not include cyclodextrins. A preferred use of such compositions is administration of the active principle by inhalation.

In a preferred embodiment of the disclosure, the pharmaceutical composition includes or consists of AT1-7 **105** at an active concentration (i.e., based on the peptide, without taking into account counterions) in the range from 0.12 to 12 mg/mL, preferably at 1 mg/mL, PS80 : AT1-7 in a molar ratio of about 0.2:1 to 3:1 , preferably at a 0.45:1 or 2:1 molar ratio, and trehalose to confer isotonicity to the solution, (e.g., at about 9.4 mass/V %), the balance being water as solvent. The composition may be substantially in the isotonic range described above. The pH of the solution may be between 4.5 to 5.5, for example at about 5.0 (e.g., ±0.1). The pH may have been adjusted by addition of extra acetic acid or acetate. AT1-7 **105** may be preferably included as acetate salt. In some embodiments, the pharmaceutical composition may be provided as a concentrated stock (e.g., 5x), and is to be diluted before administration.

Another preferred embodiment of the disclosure is the pharmaceutical composition including or consisting of AT1-7 **105** at an active concentration (i.e., based on the peptide, without taking into account counterions) in the range from 0.12 to 12 mg/mL, preferably at 1.0 mg/mL, PS20 : AT1-7 **105** in a molar ratio of about 0.1:1 to 3:1, preferably at a 0.45:1 or 2:1 molar ratio, and trehalose for isotonicity (e.g., at about 9.4 mass/V %), the balance being water as solvent. The composition may be substantially in the isotonic range described above. The pH of the solution may be between 4.5 to 5.5, for example at about 5.0 (e.g., ±0.1). The pH may have been adjusted by addition of extra acetic acid. AT1-7 **105** may be preferably included as acetate salt. In some embodiments, the pharmaceutical composition may be provided as a concentrated stock (e.g., 5x), and is to be diluted before administration.

In some aspects of the disclosure, the pharmaceutical composition may be prepared by first contacting the hydrophilic surfactant with the solvent system under stirring, followed by the active principle (AT1-7 **105).** The non-reducing sugar may be added in any order, as long as AT1-7 **105** is added to the solvent system after the hydrophilic surfactant.

A schematic process flow for the preparation of the pharmaceutical composition according to some embodiments of the invention is shown in Figure 4. First, stock solutions of the surfactant (e.g., PS80) and the non-reducing sugar (e.g. trehalose) are prepared **(S210, S220).** Preparing the stock solutions **(S210, S220)** may include flushing the solvent system (e.g., water for injection) with nitrogen until the measured oxygen level is below a predetermined threshold, for example, less than 10 ppm, preferably less than 5 ppm, more preferably less than 2 ppm. Then, the stock of the non-reducing sugar may be prepared by adding the non-reducing sugar to the flushed water, and stirring the solution until clear. The same applies for the stock of the surfactant. The two stocks (or portions thereof) so prepared are then mixed **(S230).** The oxygen level may be checked again at this stage to be below the predetermined threshold.

The mixed solution of the non-reducing sugar and the surfactant is then filled up (**S240**) with the solvent system (e.g., water for injection) close to the final intended amount of solution, for example at about 98% of the intended final

weight of solution. During and/or after the addition of water, the solution may be flushed with nitrogen. The pH may be adjusted as needed. The filled-up solution is then tested to check the pH, the oxygen level and the osmolarity are within acceptable levels. For example, the oxygen level may be checked to be below the predetermined threshold, the pH to be in the range from 4.0 to 6.0 (e.g., at about pH 5.0), and the osmolarity may be in a suitable isotonic range, such as from 250 to 350 mosmol/Kg, preferably 270 to 330 mosmol/Kg.

The fill up **(S240)** may be conducted in a series of separate steps. For example, the solution may be brought at a first weight (e.g., about 80%) of the final total weight in a first fill up step **(S242),** and the pH may be adjusted a first time to about 5.0 (e.g., ±0.1). Then, in a second fill up step **(S244)** the solution may be brought at a second weight greater than the first weight (e.g., about 98%) of the final total weight, and the pH may be adjusted again to be about 5.0 (e.g., ±0.1). In some embodiments, more than two fill-up steps may be performed.

After the physicochemical tests described above, a portion of the filled-up solution may be finished **(S250),** for example to be used as diluting solution, if needed. For example, finishing **(S250)** may include filtering, autoclaving, filling and crimping, operations with which a skilled person is well familiar. The filtering, for example, may be performed with a 0.22 μm filter. The active principle (AT1-7 **105**) may then be added **(S260),** for example by dissolving it in another portion of the filled-up solution produced from the filling step **(S240),** stirring until the solution is clear. Upon dissolution of the active principle, the composition may be brought to the final desired volume with some of the diluting solution, and the physicochemical properties may be tested again, to check the parameters are still within the reference ranges mentioned above (e.g., pH 4.0-6.0, preferably 5.0 ±0.1; oxygen level < 10 ppm, preferably <5 ppm, most preferably < 2 ppm; osmolarity 250-350 mosmol/Kg, preferably 270-330 mosmol/Kg). The tested solution may then be finished **(S270),** in a similar fashion as described before for the diluting solution. Clearly, in some embodiments, the preparation of the diluting solution **(S250)** may be omitted, and AT1-7 **105** may be added **(S260)** directly at the intended final concentration of use.

It will be apparent that the above method may be adjusted by the skilled person according to the desired product requirements. For example, the concentrations of the stocks and the final solution may be adjusted according to whether the composition is to be prepared ready for inhalation (e.g., 1x) or concentrated to be diluted before use (e.g., 5x).

Some aspects of the disclosure are also concerned with a dried powder obtainable from any one of the pharmaceutical compositions disclosed herein. In particular, the dried powder may be obtained by drying, preferably spray-drying or lyophilizing, any one of the disclosed pharmaceutical compositions. Spray-drying and freeze-drying processes are in general known to the skilled person, and various conventional types of spray-drying and freeze drying are adequately described in the state of the art, e.g. in relevant textbooks. In general freeze-drying processes comprise three stages, namely freezing, primary drying and secondary drying. During freezing the solution to be dried is converted into a solid state. The freezing is performed below a critical temperature which is the collapse temperature (Tc) for amorphous products. The freezing may also comprise at least one annealing step. During the primary drying the frozen solvent is sublimated under reduced pressure, resulting in a dry, structurally intact product. The primary drying is performed by controlling the shelf temperature (Ts) and the chamber pressure (Pc). The secondary drying is performed to remove residual moisture still bound to the product after the primary drying. While the composition is dried until a powder is obtained, the powder needs not be completely moisture free. Indeed, a certain amount of solvent (water) may remain in the powder, to facilitate subsequent reconstitution of the pharmaceutical composition. For example, the powder after the primary drying (or the secondary drying if performed) may have approx. from 0.3 to 1% of residual moisture. The residual moisture may be measured by Karl-Fischer titration using an oven to sublime residual water.

In some embodiments, it may be advantageous to store the compositions in dried powder form, for storage stability and transport. In some embodiments, the non-reducing sugar may act as a carrier for the composition, so that no additional vehicle is needed. For example, trehalose being used as the non-reducing sugar, the pharmaceutical composition (and hence the dried powder obtained therefrom) may further include only AT1-7 **105** and the hydrophilic surfactant in addition to the solvent system (traces or small amounts of which may remain as moisture in the dried powder).

In some embodiments, the pharmaceutical composition may be reconstituted from the dried powder by re-adding a suitable solvent system. For example, the dried powder may be suspended and then re-solubilized in a suitable volume of water for injection, to reconstitute any one of the pharmaceutical compositions disclosed herein. The volume of reconstituting solvent may be selected so as to achieve the desired concentration of the active principle. The reconstitution is preferably performed at or close to the intended time of administration in order to avoid any contaminations with microbes. The skilled person is familiar with the handling of pharmaceutical compositions for reconstitution and reconstituted solutions.

In some alternative embodiments, the dried powder itself may be used to administer the active principle, for example via dry-powder inhalation. In some yet alternative embodiments, suitable propellants (e.g., hydrofluorocarbons) may be added to the compositions, which may be used for administration of the active principle via metered-dose inhalers. Such propellants are usually not necessary in compositions to be used with nebulisers. Therefore, a skilled person would readily see that a composition for inhalation via metered-dose inhalers will contain a propellant, while a composition for inhalation by nebulisation would not contain the propellant.

In some aspects, the disclosure is also concerned with kits for administering AT1-7 **105** by inhalation. For

example, the kit may comprise (or consist of) the dried powder obtainable by drying any one of the pharmaceutical compositions disclosed herein, and a suitable volume of reconstituting solvent provided separately from the dried powder. For example, the kit may comprise (or consist of) a first container (e.g., a vial, a bottle, or a holder, etc.) containing the dried powder, and a second container (e.g., another vial, bottle, holder, or a separate compartment of the same vial, bottle, or holder) containing the reconstituting solvent (e.g., water for injection). Optionally, the kit may include a suitable device to facilitate inhalation, such as a nebuliser, and/or consumable parts thereof, such as nebulisation cups. Suitable examples thereof include mini-nebuliser devices with a pore size in the range 1 $\mu$m to 5 $\mu$m, such as an MNeb device with a 3.6 $\mu$m pore size. Instructions for a user as to how to reconstitute the pharmaceutical composition and/or how to use the composition with the nebuliser may optionally be included. Alternatively, the kit may include the dried powder, and a dry-powder inhaler.

In some alternative embodiments, the kit may directly provide the pharmaceutical composition, as a liquid. For example, the kit may include any one of the pharmaceutical compositions disclosed herein, and the nebuliser (or consumable parts thereof, such as the nebulisation cups). The pharmaceutical composition may be provided at the concentration of use, or to be diluted, for example with an accompanying diluting solution. In such cases, the kit may include the concentrated pharmaceutical composition, the diluting solution, and, optionally, the nebulisers (or consumable parts thereof). In some alternative embodiments, in which the pharmaceutical composition includes a propellant, the kit may include the pharmaceutical composition and a metered-dose inhaler.

The pharmaceutical compositions and the kits here disclosed are suitable to administer by inhalation AT1-7 to a subject in need thereof. Administration by inhalation via nebulisation is a particularly preferred route. Therefore, the invention is also concerned, in some aspects, with the use of such compositions as medicaments, in particular for treating conditions requiring administration of AT1-7. The conditions treated may be systemic or local conditions which particularly benefit from the vasodilatory and/or immunomodulatory action of direct inhalation of AT1-7. Exemplary conditions suitable to be treated with the disclosed compositions include respiratory diseases. In some preferred embodiments, the condition treated is one or more of acute respiratory distress syndrome (ARDS), bronchial asthma, chronic obstructive pulmonary disease, cystic fibrosis, berylliosis, and sarcoidosis.

Furthermore, it is possible that the pharmaceutical compositions as a sterile presentation may be used for the treatment of ophthalmic conditions, possibly RAS-related, , such as glaucoma, diabetic retinopathy, age-related macular degeneration, and/or retinopathy in premature newborns. Furthermore, the compositions may be used to reduce intraocular pressure in the eye.

As a still further option, the composition may be administered topically in the nose, (e.g., as nose drops). For example, the nose is a site of entry of SARS-CoV-2 and also an organ expressing ACE2. Therefore, administration of the composition could alleviate known COVID-19 symptoms such as loss of smell and taste.

The invention will now be further described by the following examples.

## Examples

### Chemicals

AT1-7 was obtained from company ABX as triacetate salt, in powder form (as lyophilized powder). Masses of AT1-7 indicated in the examples below refer to the peptide as weighted, including the counterion. The amount of "net" peptide is about 83% of the indicated weight.

PS80 and PS20 were purchased from company Croda as super refined qualities, and used as received.

Trehalose ($\alpha$, $\alpha$-Trehalose Dihydrate, High Purity (low Endotoxin) NF, EP, JP) was purchased from Pfanstiehl, and used as received.

NaCl was purchased from Bbraun as sterile isotonic solution (0.9 %), and used as received.

Glycine was purchased from Ajinomoto, and used as received.

Human albumin was purchased from CSL-Behring, and used as received.

HP$\beta$CD (Cavitron w7HP7 Pharma) was purchased from Ashland and used as received.

### Preparation of the solutions/samples

Solutions for dynamic light scattering measurements were prepared according to the procedure described above. The pyrex (7740 - type S10-G-10) measuring cuvettes were prerinsed with water for injection, dried with acetone. and the outer walls were wiped with a suitable fibre-free textile. Samples (3 mL) were filled into the cuvettes for the DLS measurements, when necessary, after sterile filtration with 0.2$\mu$m filters.

**Dynamic light scattering Measurements**

Dynamic light scattering measurements were performed with a Delsa™ Nano C zeta particle analyser by Beckman Coulter, particle size starting from 0.6 nm- 1 µm, dual 30mW laser, setting a scattering angle of 165°; attenuator 1 at 65.58%; temperature at 20,0 °C; timedomain (TD) as a correlation method; and a pinhole size of 50 µm. Optimal position of the cell was determined as part of the calibration instrument according to the manufacturer's instructions. Unless otherwise stated, samples were measured freshly prepared. Particle diameters are considered the median values (d50) of the intensity distributions. The distribution shape is further estimated by the d10 and d90 values (intensity basis). Intensity distributions were also used as a basis for the calculation of the polydispersity indexes (PDIs). Tabulated values are averages of results obtained from three measurements performed on the same sample. Values in brackets are considered to fall outside of the interval of reliability of the instrument. They are nevertheless reported as providing information on the presence of aggregates.

**Experiment 1. Solubilization of AT1-7 by hydrophilic surfactants**

Solubilization of AT1-7 in presence of PS80 was investigated by preparing and measuring a series of solutions at constant concentration of AT1-7 (1.2 mg/mL, $1.1x10^{-6}$ mol/L) and different molar ratios of PS80, as reported in Table **4.** Representative differential intensity distributions (left axis) for individual runs obtained for Reference Example 1.2, Reference Example 1.3 and Reference Example 1.5 are illustrated in Figures **5A** to **5C,** respectively. Also plotted in Figures **5A** to **5C** are the cumulative intensity distributions (right axis) resulting from the measured normalized intensity distributions.

**Table 4.** Solubilization of AT1-7 by PS80

| Reference Example N. | PS80 (mg/mL) | PS80 (mol/mL) | AT1-7 : PS80 (mol:mol) | d50 (nm, intensity) | PDI |
|---|---|---|---|---|---|
| 1.1 | - | - | - | 633.6 | 0.316 |
| 1.2 | 0.35 | $2.7x10^{-7}$ | 1:0.2 | 12.8 | 0.081 |
| 1.3 | 3.5 | $2.7x10^{-6}$ | 1: 2.0 | 11.0 | 0.093 |
| 1.4 | 12 | $9.2x10^{-6}$ | 1: 8.2 | 10.1 | 0.057 |
| 1.5 | 35 | $2.7x10^{-5}$ | 1: 24 | 9.4 | 0.040 |

As illustrated in Figures **5A-C,** even a molar ratio of AT1-7:PS80 of 1:0.2 produces samples with a significantly smaller diameter than without surfactant, which indicates suppression of larger aggregate formation.

**Experiment 2. Effect of osmolytes on particle load**

Different types of osmolytes (inorganic salts, amino acids, non-reducing sugars, and proteins) were tested at the respective isotonic concentrations to prepare isotonic solutions of AT1-7. A concentration for AT1-7 of 1.8 mg/mL (as weighted, $1.7 x10^{-6}$ mol/L) was selected, as potentially relevant for therapeutic applications. The effect of the osmolytes was investigated on solutions of AT1-7 in plain water for injection (WFI), as well as in the presence of 0.1 mass/V % PS80 (1 mg/mL, 1:0.38 mol/mol AT1-7:PS80). Results for the osmolytes alone and in presence of PS80 are reported in Table **5** and Table **6,** respectively. Distribution intensities of representative runs for NaCl, glycine, albumin, and trehalose without PS80 are illustrated in Figures **6A** to **6D,** respectively. Distribution intensities of representative runs for NaCl, glycine, and trehalose with PS80 are illustrated in Figures **7A** to **7C,** respectively. Figure **8** illustrates distribution intensities for a representative run of a solution comprising isotonic HPβCD in presence of PS80 (0.1 mass/V %).

**Table 5.** Effects of osmolytes on the solubility of AT1-7 in WFI

| | Osmolite | Conc. Osmolite (mass/Vol %) | d10 | d50 (nm, intensity) | d90 | PDI |
|---|---|---|---|---|---|---|
| Reference Example 2.1 | NaCl | 0.9 | 206.3 | 453.0 | [$2.1x10^5$] | 0.563 |
| Reference Example 2.2 | Glycine | 2.2 | 311.6 | [$7.1x10^5$] | [$1.1x10^6$] | 1.671 |
| Reference Example 2.3 | Albumin | 4 | 5.4 | 55.4 | 164.0 | 0.345 |
| Reference Example 2.4 | Trehalose | 9.4 | 18.1 | $1.5*10^3$ | [$4.8x10^5$] | 0.782 |

**Table 6.** Effects of osmolytes on the solubility of AT1-7 in presence of PS80

| | Osmolite | Conc. Osmolite (mass/Vol %) | d10 | d50 | d90 | PDI |
|---|---|---|---|---|---|---|
| | | | (nm, intensity) | | | |
| Reference Example 2.5 | NaCl | 0.9 | 10.8 | 322.5 | [$2.5x10^5$] | 0.534 |
| Reference Example 2.6 | Glycine | 2.2 | 9.6 | 10.8 | [$6.3x10^3$] | 0.269 |
| Example 2.7 | Trehalose | 9.4 | 2.6 | 8.8 | 41.9 | 0.271 |
| Reference Example 2.8 | HPβCD | 25 | 10.8 | 754.6 | [$5.6x10^3$] | 0.369 |

As the above data show, the osmolytes, by themselves, do not significantly resolve the aggregation of AT1-7. Even when combined with PS80, aggregation was observed for all osmolytes with the exception of trehalose. Notably, aggregation was still observed for HPβCD even in presence of PS80. Without being bound to or being limited by any theory, it is possible NaCl or other salts may have a salting-out effect on AT1-7. Human albumin, being the "transporter protein in blood", was tested to verify the possibility of omitting the surfactant from the compositions. However, as the experiments above clearly show, human albumin was still not capable of adequately preventing aggregation of AT1-7.

Notably, for the combination of trehalose and PS80, only signals attributable to micelles by the surfactant were observed by DLS. The same effect was observed for more concentrated solutions (e.g., 5 mg/mL of net (excluding counterion) AT1-7, 0.3% m/V PS80 - AT1-7:PS80 1:0.41; 9.4% m/V trehalose). DLS (Figure **9A**) and turbidity measurements (NTU $0.4\pm0.1$, post filtration) of such concentrated solutions also did not reveal presence of aggregates. Furthermore, the experiment of Example 2.7 was repeated with mannitol (5% m/V) and sorbitol (5.4 % m/V), with a PS80:AT1-7 molar ratio of 0.57:1 (0.1% m/V PS80), with detected d50 of about 18 nm.

Surprisingly, direct comparison of intensity distributions measured in presence and absence of trehalose revealed narrower distributions in presence of trehalose than with PS80 alone. In Figure **9B** are compared the measured distributions for the solution of Example 1.2 (distribution **300),** and a solution identical to Example 1.2 but also including 9.4 wt% trehalose (distribution **302).** Clearly, distribution **302** is centred at lower diameters and narrower with respect to distribution **300.** Without being bound to or limited by any theory, our data suggests the presence of a synergistic effect between the surfactant and the non-reducing sugar in the solubilization of AT1-7.

### Experiment 3. Solutions with PS20

Solutions of isotonic trehalose (9.4 mass/V %) and AT1-7 (1.20 mg/mL as weighted, $1.1 x10^{-6}$ mol/mL), including PS20 as hydrophilic surfactant in place of PS80, were prepared and measured as described above. Water for injection was used as solvent, and no further additive or excipient was added. Results are reported in Table 7.

**Table 7.** Results for solutions including PS20

| | Surfactant | Conc. Surfactant (mass/Vol %) | AT1-7:PS mol:mol | d50 (nm, intensity) | PDI |
|---|---|---|---|---|---|
| Example 3.1 | PS20 | 0.1 | 1:0.7 | 11.0 | 0.621 |
| Example 3.2 | PS20 | 0.3 | 1:2.2 | 10.7 | 0.222 |
| Example 3.3 | PS80 | 0.1 | 1:0.7 | 10.8 | 0.488 |
| Example 3.4 | PS80 | 0.3 | 1:2.1 | 10.9 | 0.417 |

From the above experiments, it can be seen that hydrophilic surfactants such as polysorbates can be expected to successfully solubilize AT1-7.

### Experiment 4. ACE Bioassay on nebulised AT1-7

In this experiment it is checked whether AT1-7 maintains its biological activity following nebulisation. The DLS of solutions of AT1-7 (1.6 mg/mL, $1.5 x10^{-6}$ mol/mL) with PS80 (0.3 mass/V %; AT1-7:PS80 1:1.3 mol:mol) and isotonic trehalose (9.4 mass/V %) in water for injections was measured before nebulisation, and distributions such as the one in Figure **9A** were obtained (measured d50: 11.0 nm). The solutions were then nebulised with a M-Neb® device at 3.6 μm pore size (Nebutec®). The nebulised droplets were pooled for testing for biological activity using an Angiotensin-converting enzyme assay, adapted after Carmona et al., *Nature Protocols,* **2006,** *1*, 1971-1976).

In detail, ACE activity was detected by monitoring the increase in emitted fluorescence by the cleaved peptide probe Abz-FRK-Dnp-P-OH (structure illustrated below). The probe is cleaved by ACE at either side of the arginine residue, with consequent emission of fluorescence upon increased distance between the di-nitrophenyl moiety and the aminophenyl moiety. It is therefore possible to follow the increase of fluorescence in a timeresolved manner (cf. curve **310** in Figure **10**) as a measure of the ACE activity. Upon addition of a competing substrate for ACE (i.e., AT1-7 **105**), the fluorescence increase is slowed down (cf. curves **312** and **314** in Figure **10).**

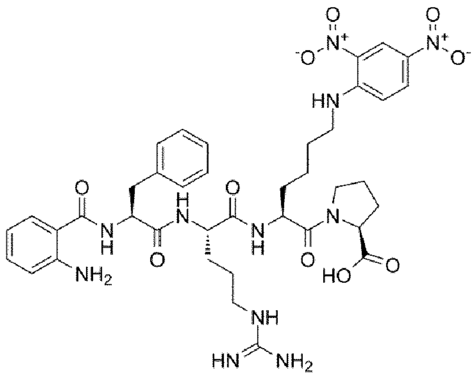

To obtain the traces in Figure 10, the following protocol was followed.

Stock solutions were prepared as follow. 0.5 mg of Abz-FRK-Dnp-P-OH (hydrochloride salt, obtained by Bachem) were dissolved in 500μL of DMSO (Molecular biology grade 100%). The concentration was adjusted according to the measured absorbance of 360nm to 1 mmol/L (target absorbance = 0.173 in DMSO). Separately, ACE (rabbit lung, 2.0 units/mg protein, from Sigma Aldrich, Prod. N. A6778) was dissolved in 1 ml per Unit activity Assay buffer (Tris base 12.1 g/L; ZnCl 1.36 mg/L; NaCl 2.92 g/mL; adjusted to pH 7.0 with NaOH 1M and HCl 1M).

Fluorescence measurements were performed in a plate reader (96-well format) with auto dispense function by BMG Labtech, Clariostar. The detection window was set at 380 to 480 nm. In each well, concentration of ACE was set at about 10μU/μL, concentration of Abz-FRK-Dnp-P-OH was set at about 4μmol/L and AT1-7 samples were diluted to aim for a concentration of 1.1 μmol/L per well. In the wells, AT1-7 was added first, followed by Abz-FRK-DnP-P-OH and finally by ACE.

The resulting fluorescence recovery traces are illustrated in Figure **10.** Trace **310** was obtained from a sample in which no AT1-7 **105** was added, and corresponds to full ACE activity; trace **312** was obtained from a sample in which AT1-7 **105** was added from a solution stored at 2-8 °C for 4 days; trace **314** was obtained from a sample in which AT1-7 **105** was added from the same solution used for trace **312** following nebulization with an M-Neb® nebulizer (3.6 μm mesh). The delay observed for the traces **312** and **314** with respect to the trace **310** indicates that AT1-7 **105,** also after nebulisation, effectively competes with the probe for ACE; furthermore, the trace **314** being almost indistinguishable (within experimental error) from the trace **312** indicates that even upon nebulisation, AT1-7 **105** maintains substantially unaltered its biological activity.

**Experiment 5. Short-term stability**

In this experiment, the stability upon short-term storage of pharmaceutical compositions (AT1-7 1.8 mg/mL as weighted, $1.6x10^{-6}$ mol/L; trehalose 9.4% m/V; PS80 0.1 % m/V or PS80 1% m/V) prepared as described above was investigated. The solutions were stored for 7 days at a temperature between 2 and 8 °C. DLS traces were measured on the solutions freshly prepared and after storage. Compositions including 1 % m/V of PS80 were yielded substantially unaltered DLS spectra (d50 freshly prepared: 13 nm; d50 after storage: 13.5 nm). Compositions including 0.1 % m/V of PS80 showed growth of a small peak at diameter below 100 nm, indicating that while lower PS80:AT1-7 molar ratios are still effective to solubilize AT1-7, higher PS80:AT1-7 molar ratios may be more effective for long term storage stability.

**List of sequences**

SEQ. ID. N. 1: ATII: Asp-Arg-Val-Tyr-Ile-His-Pro-Phe
SEQ. ID. N. 2: AT1-7: Asp-Arg-Val-Tyr-Ile-His-Pro
SEQ. ID. N. 3: ATI: Asp-Arg-Val-Tyr-Ile-His-Pro-Phe-His-Leu
SEQ. ID. N. 4: AT1-5: Asp-Arg-Val-Tyr-Ile

**Claims**

1. An aqueous isotonic pharmaceutical composition, comprising:

   the peptide Angiotensin(1-7) with a sequence according to SEQ. ID. N. 2, Asp-Arg-Val-Tyr-Ile-His-Pro, optionally accompanied by a pharmaceutically acceptable counterion, as active principle;
   a polysorbate as hydrophilic surfactant, having a hydrophilic-lipophilic balance index, HLB, value of 8 or more, being included at a molar ratio with respect to Angiotensin(1-7) of at least 0.1:1 (surfactant:Angiotensin 1-7); and
   trehalose as non-reducing sugar, included at an amount to ensure isotonicity of the pharmaceutical composition,
   wherein the pH of the pharmaceutical composition is in a range from 4.0 to 6.0.

2. The pharmaceutical composition of claim 1, wherein the active principle is accompanied by acetate as the pharmaceutically acceptable counterion.

3. The pharmaceutical composition according to claim 1 or 2, wherein the hydrophilic surfactant is selected from the group consisting of polyoxyethylene (20) sorbitan monooleate (PS80) and polyoxyethylene (20) sorbitan monolaurate (PS20), and more preferably the surfactant is polyoxyethylene (20) sorbitan monooleate (PS80).

4. The pharmaceutical composition according to any one of the preceding claims, wherein a molar ratio of the active principle to the hydrophilic surfactant is:

   in a range from 1:0.1 to 1:20, preferably in a range from 1:0.1 to 1:3, more preferably in a range from 1:0.2 to 1:0.8, even more preferably in a range from 1:0.3 to 1:0.6, most preferably in a ratio of 1:0.4;
   or
   in a range from 1:0.1 to 1:20, preferably in a range from 1:0.5 to 1:15, more preferably in a range from 1:1 to 1:4, even more preferably in a range from 1:1.5 to 1:3, most preferably in a ratio of 1:2.

5. The pharmaceutical composition according to any one of claims 1 to 4, consisting of the active principle, the non-reducing sugar, the hydrophilic surfactant, an optional pH adjusting agent, and water.

6. The pharmaceutical composition according to any one of claims 1 to 5, wherein a content of the active principle is in a range from 1.3 x$10^{-7}$ to 1.3x$10^{-5}$ mol/mL.

7. A dried powder, obtainable by drying, preferably by freeze-drying or spray-drying, the pharmaceutical composition according to any one of claims 1-6.

8. A kit for administering Angiotensin(1-7) as active principle by inhalation, the kit comprising:

   the dried powder according to claim 7; and
   water for injection for reconstituting the dried powder into the pharmaceutical composition of any one of claims 1-6, the water being provided in a separate container from the dried powder.

9. A kit for administering Angiotensin(1-7) as active principle by inhalation, the kit comprising:

   the pharmaceutical composition according to any one of claims 1 or 6; and
   a nebuliser or consumable parts therefor.

10. The composition according to any one of claims 1-6 for use as a medicament.

11. The composition for the use according to claim 10 in the treatment of a local or systemic condition in a subject requiring administration of the Angiotensin(1-7) as active principle, the composition being administered by inhalation, preferably by nebulisation, and preferably for the treatment of a respiratory condition, further preferably the respiratory condition being selected from the group consisting of acute respiratory distress syndrome (ARDS), bronchial asthma, chronic obstructive pulmonary disease, cystic fibrosis, berylliosis, sarcoidosis, and acute lung injury, the acute lung injury preferably caused by COVID-19.

12. The pharmaceutical composition for the use according to claim 10, in the treatment of a condition selected from the group consisting of glaucoma, diabetic retinopathy, age-related macular degeneration, retinopathy in premature

newborns, and elevated intraocular pressure, the composition being administered in the eye.

13. The composition for the use according to claim 10 in the treatment of loss of smell and/or taste in a subject affected by COVID-19, the composition being topically administered as nasal drops.

**Patentansprüche**

1. Wässerige isotonische pharmazeutische Zusammensetzung, umfassend:

   das Peptid Angiotensin(1-7) mit einer Sequenz gemäß SEQ ID NO: 2, Asp-Arg-Val-Tyr-Ile-His-Pro, optional in Verbindung mit einem pharmazeutisch verträglichen Gegenion, als Wirkstoff;
   ein Polysorbat als hydrophiles Tensid mit einem Hydrophil-Lipophil-Balance-Index, HLB, Wert von 8 oder mehr, das in einem Molverhältnis zu Angiotensin(1-7) von mindestens 0,1:1 (Tensid:Angiotensin(1-7)) enthalten ist; und
   Trehalose als nichtreduzierender Zucker, enthalten in einer Menge, die die Isotonie der pharmazeutischen Zusammensetzung gewährleistet,
   wobei der pH-Wert der pharmazeutischen Zusammensetzung im Bereich von 4,0 bis 6,0 liegt.

2. Pharmazeutische Zusammensetzung gemäß Anspruch 1, wobei der Wirkstoff von Acetat als pharmazeutisch verträglichem Gegenion begleitet wird.

3. Pharmazeutische Zusammensetzung gemäß Anspruch 1 oder 2, wobei das hydrophile Tensid ausgewählt ist aus der Gruppe bestehend aus Polyoxyethylen (20) Sorbitanmonooleat (PS80) und Polyoxyethylen (20) Sorbitanmonolaurat (PS20), und noch bevorzugter das Tensid Polyoxyethylen (20) Sorbitanmonooleat (PS80) ist.

4. Pharmazeutische Zusammensetzung gemäß einem der vorstehenden Ansprüche, wobei ein Molverhältnis von Wirkstoff zu hydrophilem Tensid:

   in einem Bereich von 1:0,1 bis 1:20, vorzugsweise in einem Bereich von 1:0,1 bis 1:3, noch bevorzugter in einem Bereich von 1:0,2 bis 1:0,8, am meisten bevorzugt in einem Bereich von 1:0,3 bis 1:0,6, am meisten bevorzugt in einem Verhältnis von 1:0,4 ist;
   oder
   in einem Bereich von 1:0,1 bis 1:20, vorzugsweise in einem Bereich von 1:0,5 bis 1:15, noch bevorzugter in einem Bereich von 1:1 bis 1:4, am meisten bevorzugt in einem Bereich von 1:1,5 bis 1:3, am meisten bevorzugt in einem Verhältnis von 1:2 ist.

5. Pharmazeutische Zusammensetzung gemäß einem der Ansprüche 1 bis 4, bestehend aus dem Wirkstoff, dem nichtreduzierenden Zucker, dem hydrophilen Tensid, einem optionalen pH-Einstellmittel und Wasser.

6. Pharmazeutische Zusammensetzung gemäß einem der Ansprüche 1 bis 5, wobei der Gehalt an Wirkstoff in einem Bereich von $1{,}3 \times 10^{-7}$ bis $1{,}3 \times 10^{-5}$ mol/ml liegt.

7. Getrocknetes Pulver, erhältlich durch Trocknen, vorzugsweise durch Gefriertrocknen oder Sprühtrocknen, der pharmazeutischen Zusammensetzung gemäß einem der Ansprüche 1 bis 6.

8. Kit zur Verabreichung von Angiotensin(1-7) als Wirkstoff durch Inhalation, wobei das Kit umfasst:

   das getrocknete Pulver gemäß Anspruch 7; und
   Wasser zur Injektion zur Rekonstitution des getrockneten Pulvers zu der pharmazeutischen Zusammensetzung gemäß einem der Ansprüche 1 bis 6, wobei das Wasser in einem vom getrockneten Pulver getrennten Behälter bereitgestellt wird.

9. Kit zur Verabreichung von Angiotensin(1-7) als Wirkstoff durch Inhalation, wobei das Kit umfasst:
   die pharmazeutische Zusammensetzung gemäß einem der Ansprüche 1 bis 6; und
   einen Vernebler oder Verbrauchsteile dafür.

10. Zusammensetzung gemäß einem der Ansprüche 1 bis 6 zur Verwendung als Arzneimittel.

11. Zusammensetzung zur Verwendung gemäß Anspruch 10 bei der Behandlung einer lokalen oder systemischen Erkrankung bei einem Patienten, der die Verabreichung von Angiotensin(1-7) als Wirkstoff benötigt, wobei die Zusammensetzung durch Inhalation, vorzugsweise durch Vernebelung, verabreicht wird, und vorzugsweise zur Behandlung einer Atemwegserkrankung, wobei die Atemwegserkrankung ferner vorzugsweise ausgewählt ist aus der Gruppe bestehend aus akutem Atemnotsyndrom (ARDS), Bronchialasthma, chronisch obstruktiver Lungenerkrankung, zystischer Fibrose, Berylliose, Sarkoidose und akuter Lungenverletzung, wobei die akute Lungenverletzung vorzugsweise durch COVID-19 verursacht wird.

12. Pharmazeutische Zusammensetzung zur Verwendung gemäß Anspruch 10 bei der Behandlung einer Erkrankung, ausgewählt aus der Gruppe bestehend aus Glaukom, diabetischer Retinopathie, altersbedingter Makuladegeneration, Retinopathie bei Frühgeborenen und erhöhtem Augeninnendruck, wobei die Zusammensetzung ins Auge verabreicht wird.

13. Zusammensetzung zur Verwendung gemäß Anspruch 10 bei der Behandlung von Geruchs- und/oder Geschmacksverlust bei einem von COVID-19 betroffenen Patienten, wobei die Zusammensetzung topisch als Nasentropfen verabreicht wird.

**Revendications**

1. Composition pharmaceutique aqueuse isotonique comprenant :

   le peptide angiotensine (1-7) ayant la séquence décrite dans SEQ ID NO : 2, Asp-Arg-Val-Tyr-Ile-His-Pro, éventuellement en association avec un contre-ion pharmaceutiquement acceptable, en tant que agent actif ;
   un polysorbate en tant qu'agent tensioactif hydrophile ayant un indice d'équilibre hydrophile-lipophile (HLB) d'au moins 8, présent dans un rapport molaire par rapport à l'angiotensine (1-7) d'au moins 0,1:1 (agent tensioactif:angiotensine (1-7)) ; et
   du tréhalose en tant que sucre non réducteur, présent en une quantité garantissant l'isotonie de la composition pharmaceutique,
   le pH de la composition pharmaceutique se situant dans la plage de 4,0 à 6,0.

2. Composition pharmaceutique selon la revendication 1, dans laquelle le agent actif est accompagné d'acétate en tant que contre-ion pharmaceutiquement acceptable.

3. Composition pharmaceutique selon la revendication 1 ou 2, dans laquelle l'agent tensioactif hydrophile est choisi dans le groupe constitué par le polyoxyéthylène (20) monooléate de sorbitane (PS80) et le polyoxyéthylène (20) monolaurate de sorbitane (PS20), et de préférence encore l'agent tensioactif polyoxyéthylène (20) monooléate de sorbitane (PS80).

4. Composition pharmaceutique selon l'une des revendications précédentes, dans laquelle le rapport molaire entre le agent actif et l'agent tensioactif hydrophile :
   se situe dans une plage de 1:0,1 à 1:20, de préférence dans une plage de 1:0,1 à 1:3, plus préférablement dans une plage de 1:0,2 à 1:0,8, encore plus préférablement dans une plage de 1:0,3 à 1:0,6, et le plus préférablement dans un rapport de 1:0,4 ;
   ou
   dans une plage de 1:0,1 à 1:20, de préférence dans une plage de 1:0,5 à 1:15, plus préférablement dans une plage de 1:1 à 1:4, le plus préférablement dans une plage de 1:1,5 à 1:3, et le plus préférablement dans un rapport de 1:2.

5. Composition pharmaceutique selon l'une des revendications 1 à 4, constituée du agent actif, du sucre non réducteur, de l'agent tensioactif hydrophile, d'un agent d'ajustement du pH facultatif et d'eau.

6. Composition pharmaceutique selon l'une des revendications 1 à 5, dans laquelle la teneur en agent actif se situe dans une plage de $1,3 \times 10^{-7}$ à $1,3 \times 10^{-5}$ mol/ml.

7. Poudre séchée, pouvant être obtenue par séchage, de préférence par lyophilisation ou séchage par atomisation, de la composition pharmaceutique selon l'une des revendications 1 à 6.

8. Kit pour l'administration d'angiotensine(1-7) en tant que agent actif par inhalation, le kit comprenant :

la poudre séchée selon la revendication 7 ; et
de l'eau pour injection destinée à la reconstitution de la poudre séchée en la composition pharmaceutique selon l'une des revendications 1 à 6, l'eau étant fournie dans un récipient séparé de la poudre séchée.

**9.** Kit pour à l'administration d'angiotensine (1-7) en tant que agent actif par inhalation, ledit kit comprenant :
la composition pharmaceutique selon l'une des revendications 1 à 6 ; et un nébuliseur ou des pièces de rechange pour celui-ci.

**10.** Composition selon l'une des revendications 1 à 6 destinée à être utilisée comme médicament.

**11.** Composition destinée à être utilisée selon la revendication 10, dans le traitement d'une affection locale ou systémique chez un patient nécessitant l'administration d'angiotensine (1-7) en tant que agent actif, ladite composition étant administrée par inhalation, de préférence par nébulisation, et de préférence pour le traitement d'une affection respiratoire, l'affection respiratoire étant en outre de préférence choisie dans le groupe constitué du syndrome de détresse respiratoire aiguë (SDRA), de l'asthme bronchique, de la bronchopneumopathie chronique obstructive, de la mucoviscidose, de la bérylliose, de la sarcoïdose et de la lésion pulmonaire aiguë, la lésion pulmonaire aiguë étant de préférence causée par la COVID-19.

**12.** Composition pharmaceutique destinée à être utilisée selon la revendication 10, dans le traitement d'une affection choisie dans le groupe constitué du glaucome, de la rétinopathie diabétique, de la dégénérescence maculaire liée à l'âge, de la rétinopathie du prématuré et l'hypertension intraoculaire, ladite composition étant administrée dans l'œil.

**13.** Composition destinée à être utilisée selon la revendication 10, dans le traitement de la perte de l'odorat et/ou du goût chez un patient atteint de la COVID-19, ladite composition étant administrée par voie topique sous forme de gouttes nasales.

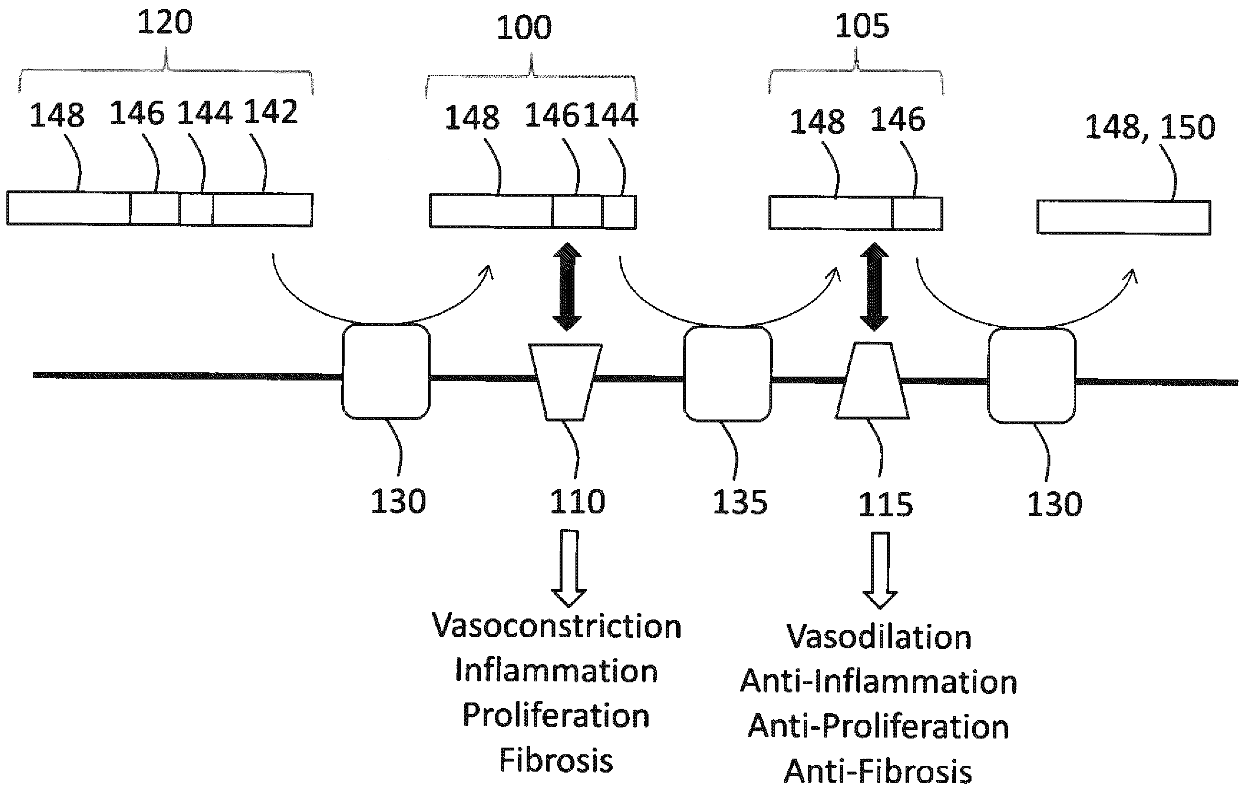
120
148 146 144 142
100
148 146144
105
148 146
148, 150
130
110
135
115
130
Vasoconstriction
Inflammation
Proliferation
Fibrosis
Vasodilation
Anti-Inflammation
Anti-Proliferation
Anti-Fibrosis

FIGURE 1A

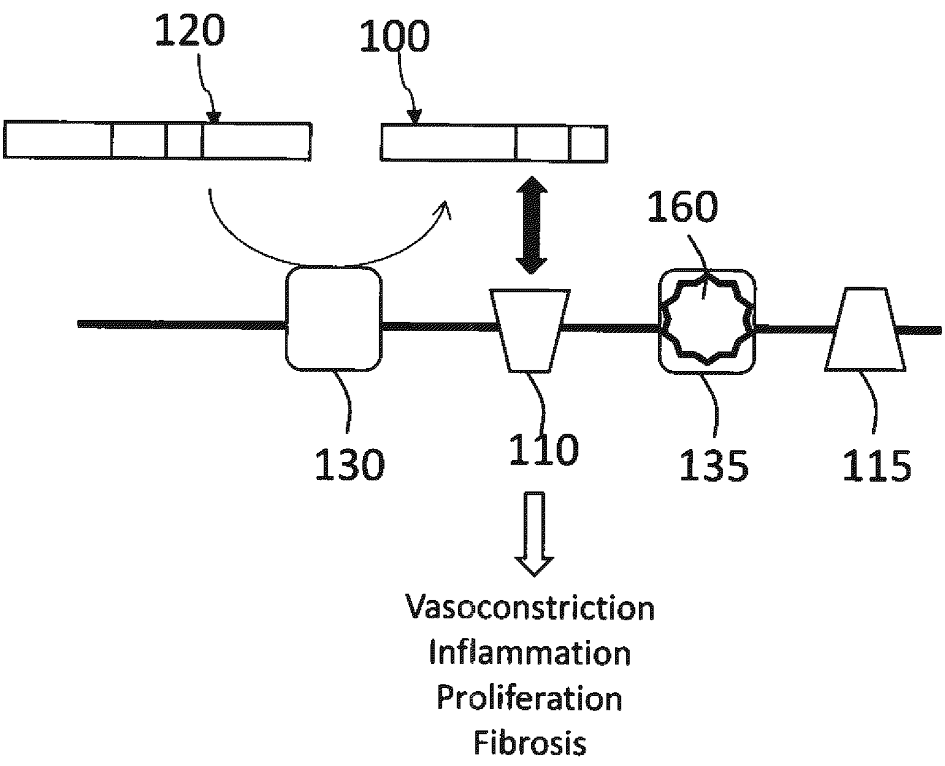


FIGURE 1B

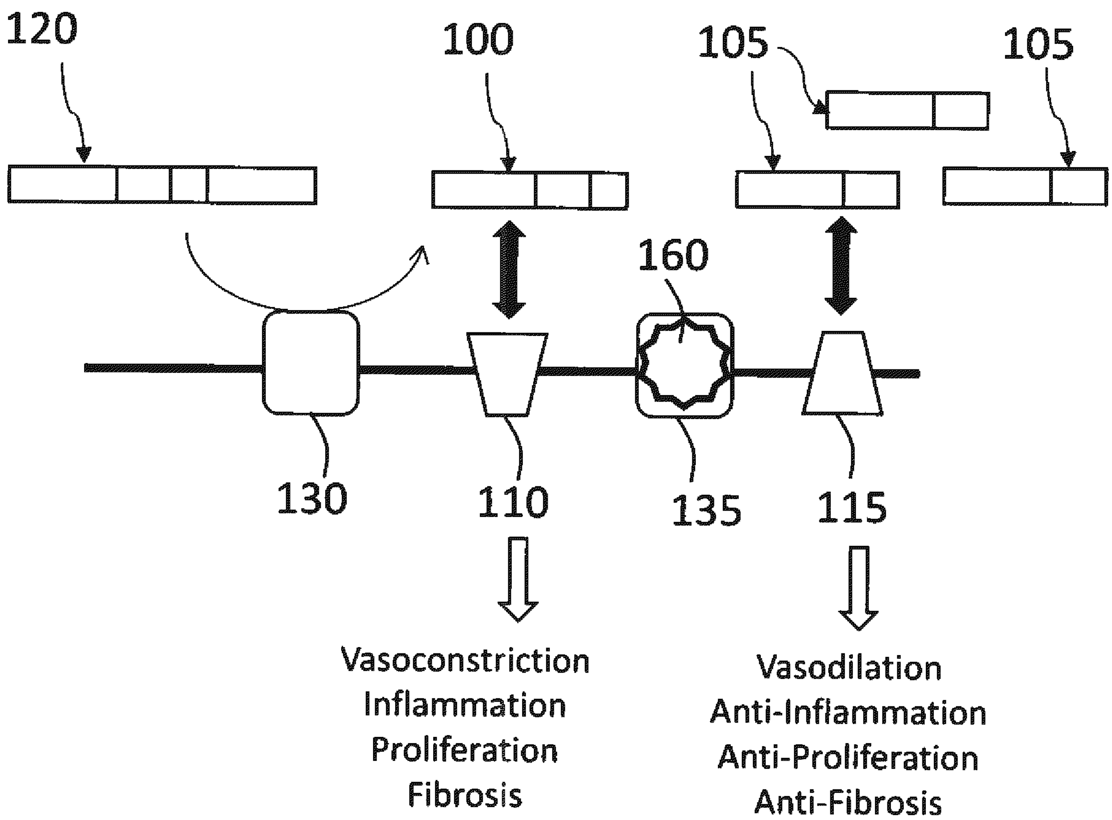


FIGURE 1C

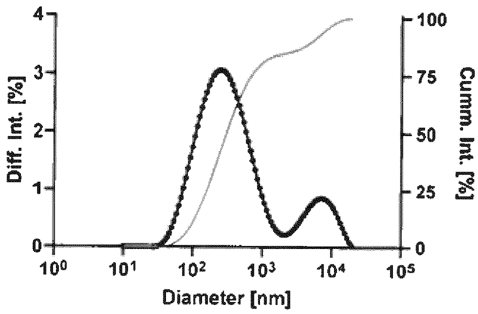


FIGURE 2A

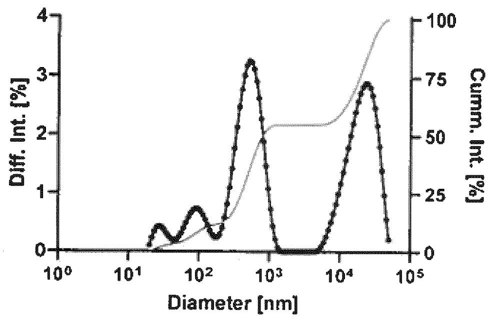


FIGURE 2B

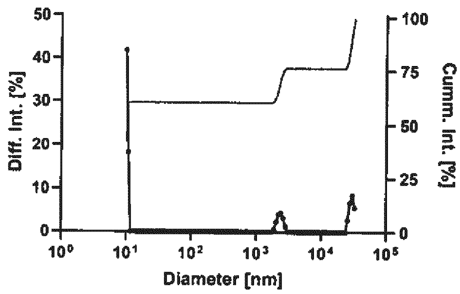


FIGURE 3A

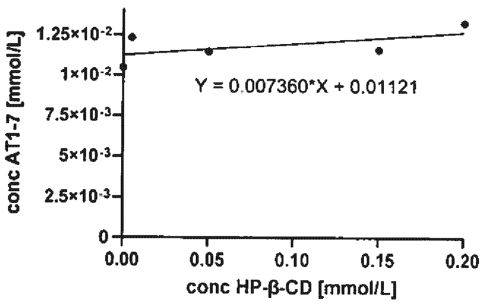


FIGURE 3B

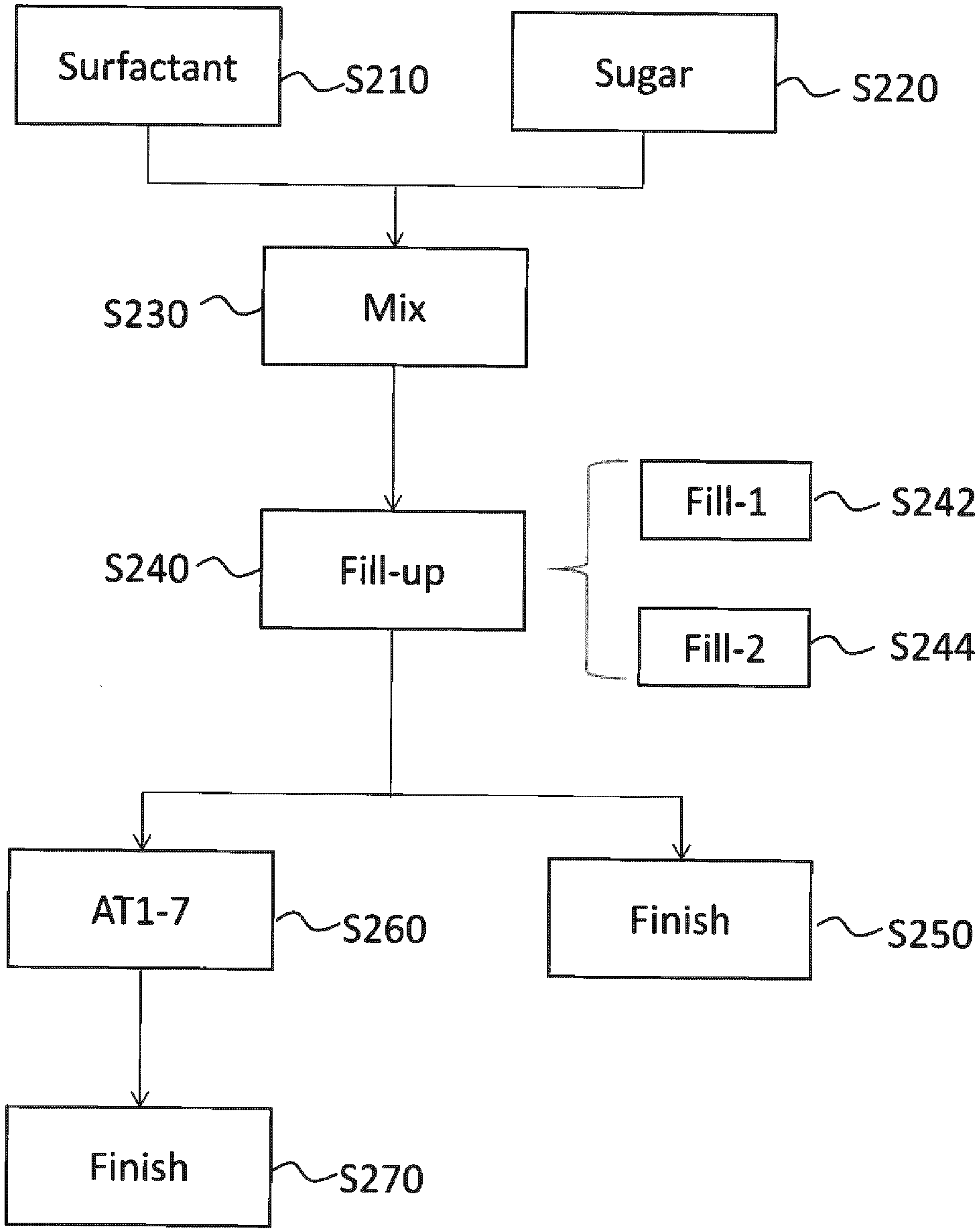
Surfactant
S210
Sugar
S220
S230
Mix
S240
Fill-up
Fill-1
S242
Fill-2
S244
AT1-7
S260
Finish
S250
Finish
S270

FIGURE 4

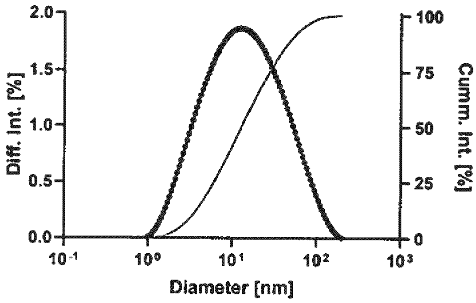


FIGURE 5A

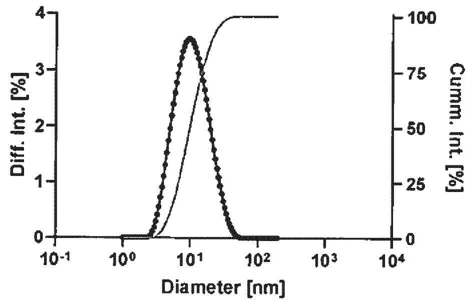


FIGURE 5B

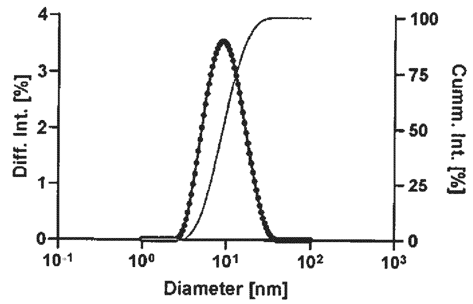


FIGURE 5C

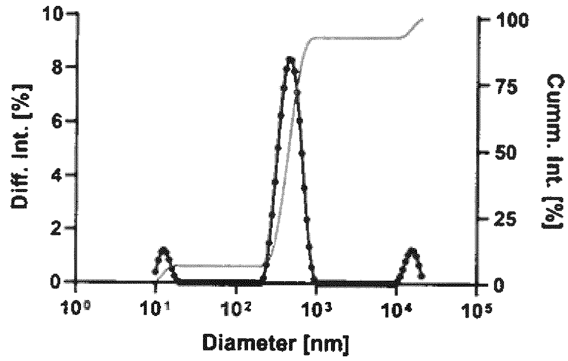


FIGURE 6A

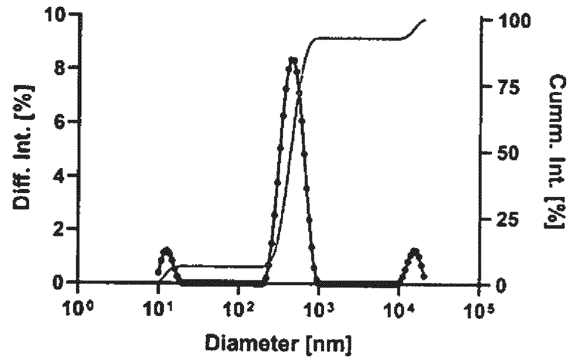


FIGURE 6B

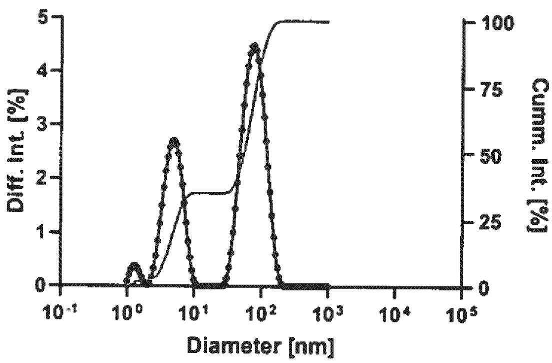


FIGURE 6C

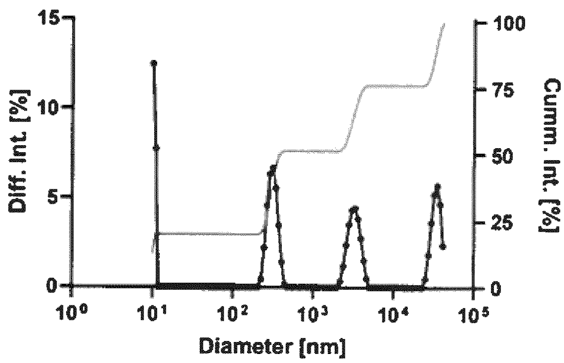


FIGURE 6D

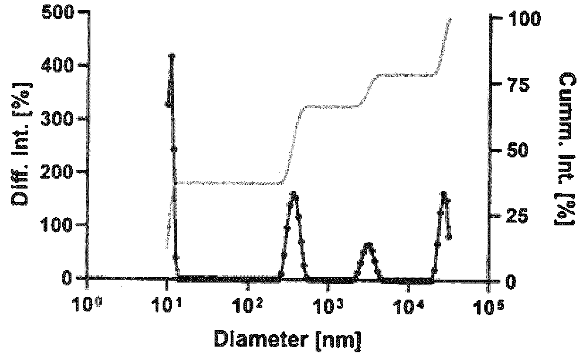


FIGURE 7A

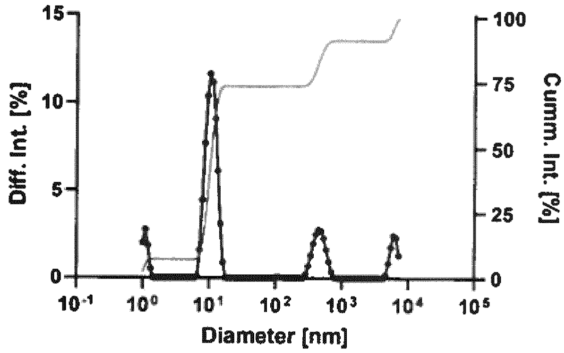


FIGURE 7B

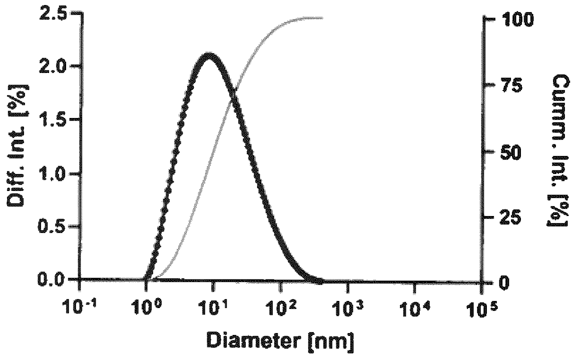


FIGURE 7C

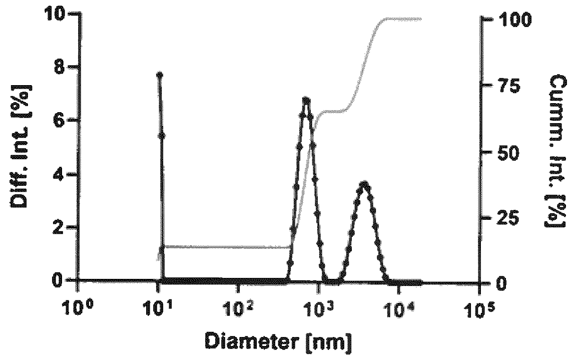


FIGURE 8

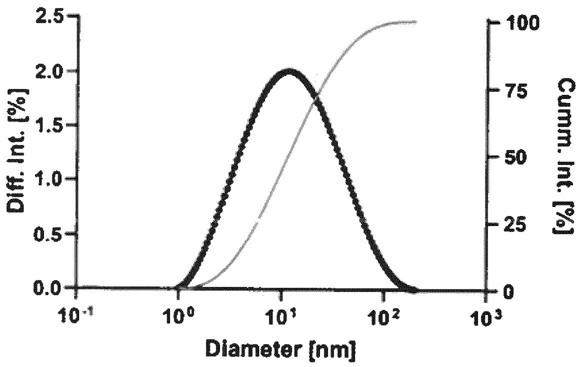


FIGURE 9A

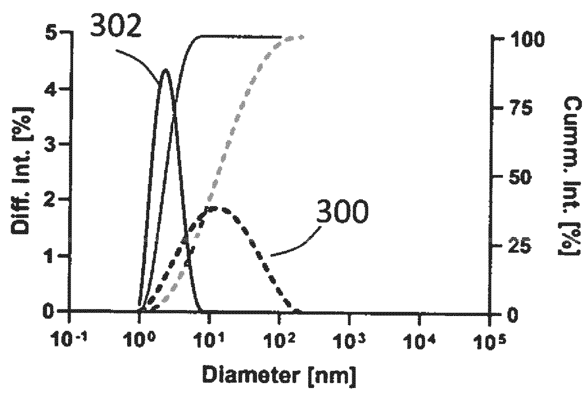


FIGURE 9B

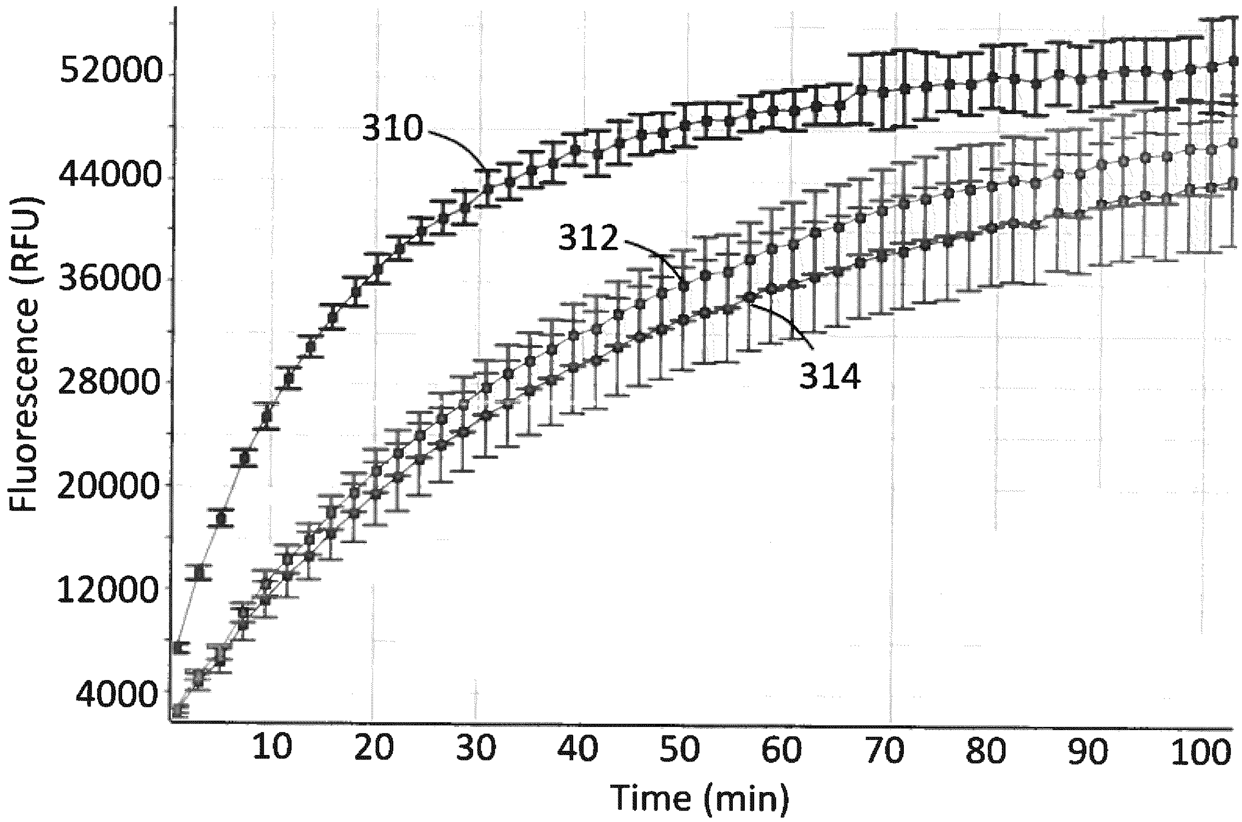
52000
44000
36000
28000
20000
12000
4000
Fluorescence (RFU)
10
20
30
40
50
60
70
80
90
100
Time (min)
310
312
314

FIGURE 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2356995 A2
- EP 1450842 B1
- EP 2264048 B1
- WO 2007000036 A2, Santos, R.A.S.
- DE 2846200 A1
- WO 0155176 A2
- WO 2021183863 A1
- WO 2009055651 A1
- WO 2022094025 A1


### Non-patent literature cited in the description


- **HOLAPPA et al.** *The Open Ophthalmology Journal*, 2017, vol. 11, 122-142
- **KUIPERS et al.** *Peptides*, 2019, vol. 112, 78-84
- **MASCOLO et al.** *Frontiers in Pharmacology*, 2021, vol. 12, 667254
- **NOZATO et al.** *Clinical Science*, 2019, vol. 133, 2005-2018
- **VAAJANEN et al.** *Investigative Ophthalmology and Visual Science*, 2008, vol. 49, 2557-2562
- **ZHOU et al.** *Nature*, 2020, vol. 579, 270-273
- **CHATTERJEE, B.** ; **THAKUR, S.S.** *RSC Advances*, 2020, vol. 10, 39808-39813
- **KUBA et al.** *Nature Medicine*, 2005, vol. 11, 875-879
- **LIU et al.** *J. Clin. Lab. Anal.*, 2021, vol. 35, e23789
- **NI et al.** *Critical Care*, 2020, vol. 24, 422
- **PEIRÓ C.** ; **MONCADA S.** *Circulation*, 2020, vol. 141, 1665-1666
- **MAGALHAES, G. S. et al.** *Frontiers in Physiology*, 2020, vol. 11, 73
- **CAO Y. et al.** *Laboratory Investigation*, 2019, vol. 99, 1770-1783
- **SUPÉ et al.** *British Journal of Pharmacology*, 2016, vol. 173, 1618-1628
- Bestimmung des optimalen Zeitfensters für die therapeutische Anwendung von Angiotensin-(1-7) beim akuten Atemnotsyndrom bei der Ratte. **SUPÉ**. Doctoral Dissertation. Freien Universität, 2018
- **KHAJEHPOUR S.** ; **AGHAZEDEH-HABASHI A.** *J. Pharmacol. Exp. Ther.*, 2021, vol. 377, 64-74
- **MAGALHÃES et al.** *Immunobiology*, 2020, vol. 225, 151957
- **HIGUCHI T.** ; **CONNORS**. *Advances in Analytical Chemistry and Instrumentation*, 1965, 177-212
- **GADHAVE A.** *International Journal of Science and Research*, 2014, vol. 3, 573-575